Europäisches Patentamt

(19) European Patent Office

Office européen des brevets

(11) Publication number: **0 276 153**
**A2**

## (12) EUROPEAN PATENT APPLICATION

(21) Application number: **88300483.0**

(22) Date of filing: **21.01.88**

(51) Int. Cl.⁴: **A 61 B 17/58**

(30) Priority: **22.01.87 DE 3701765**

(43) Date of publication of application:
**27.07.88 Bulletin 88/30**

(84) Designated Contracting States:
**BE ES FR GB IT NL**

(71) Applicant: **ETHICON INC.**
**U.S. Route 22**
**Somerville New Jersey 08876 (US)**

(72) Inventor: **Schilder, Lothar**
**Lawaetzweg 11**
**D-2000 Hamburg 20 (DE)**

(74) Representative: **Jones, Alan John et al**
**CARPMAELS & RANSFORD 43 Bloomsbury Square**
**London, WC1A 2RA (GB)**

(54) **Bone screw.**

(57) The invention relates to a bone screw with a threaded part and a screw head, characterized in that a head plate (3) is provided between shank (5) and screw head (1) and on whose centre is located said screw head and that the transition zone (2) between the head plate and the screw head is constructed as a predetermined shearing zone, which shears at a torque corresponding to 0.2 to 0.4 times the pulling off force for separating head plate (3) and shank (5).

EP 0 276 153 A2

**Description**

## BONE SCREW

For stabilizing bone fractures, for joining together bone fragments, in the implantation of joint prostheses and in numerous other surgical applications, bone parts are fixed by means of bone screws, either alone or in conjunction with a bone plate and such as are e.g. described in DE-OS 33 01 298. These known bone screws are generally made from metal and must be reoperatively explanted after a certain time, which causes additional stress to the patient and further costs to the public.

The problem of the present invention is to obviate these disadvantages and to use fixing systems made from resorbable plastics, which make any reoperation unnecessary.

Resorbable plastics based on polydioxanone, polylactide and polyglycolides are known per se and have been proposed as implants for the most varied uses. However, as the strength of resorbable plastics is limited, many difficulties are encountered in making bone screws from such resorbable plastics. A bone screw of this type made from resorbable plastic must have an adequate strength over a period of time and also damage during the fitting of such screws must be avoided. When fitting such a bone screw, the surgeon must ensure that it is not turned in too far, because this could lead to damage to the screw head. Alternatively, if the bone screw was turned in too vigorously, there would be an excessive static force between screw head and screw shank, which would lead to separation of the screw head from the shank after implantation in the case of a resorbable material.

The further problem set by the invention is to so improve a bone screw comprising a threaded part, whose shank is terminated in one piece in a screw head, that excessive turning in and therefore damage to the screw head is avoided and also there is no excessive static force between the screw head and the screw shank, which would lead to the separation of head from shank after implantation in the case of a resorbable material.

For solving this problem, a bone screw of the aforementioned type is proposed, which is characterized in that a head or cover plate is provided between shank and screw head and on whose centre is located the screw head and that the transition zone between the head plate and the screw head is constructed as a predetermined or desired shearing zone, which shears at a torque corresponding to 0.2 to 0.4 times the pulling off force for separating head plate and shaft.

Further preferred embodiments are referred to in the subclaims.

It has surprisingly been found that in the case of the inventive construction of the bone screw with head plate and screw head, a type of "double head" is formed, which firstly comprises the screw head on which acts the screwdriver and secondly the head plate, which keeps the bone plates in position. Thus, in the case of the desired shearing of the screw head, no damage occurs to the head plate and retains its function as a screw head plate. As a result of a corresponding dimensioning of the shearing zone, the surgeon can screw in the inventive bone screw until the screw head shears. The head plate is then left as the screw head of the bone screw, a precisely defined maximum static force prevailing between the head plate now forming the head and the shank.

This embodiment makes it possible to adapt the force to be applied on screwing in the bone screw in relationship to the screw thread, which must have an adequate core strength adapted to the consistency of the bone.

Preferably the predetermined shearing zone has a cross-sectional surface 60 to 80% less than that of the screw shank.

In order in certain cases to permit a removal of the bone screw, two or more bores for receiving the pins of a box spanner should be provided in the outer peripheral region of the head plate.

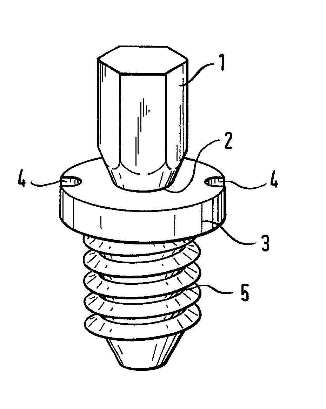

The invention is described in greater detail hereinafter relative to a non-limitative embodiment and the attached drawing, which is a perspective view of the inventive bone screw.

The resorbable material bone screw shown in the drawing comprises a threaded shank 5, whose thread depth and pitch can be adapted to the intended use. To shank 5 is connected a head plate 3, which serves as a support on the bone plate. Head plate 3 carries the screw head 1, which in the present case is constructed as a hexagonal stump and fits into a corresponding hollow spanner. A slotted screw head can be provided in place of a hexagonal stump. It is important that the transition zone 2 between screw head 1 and head plate 3 is preferably tapered or in some other way constructed as the predetermined shearing zone.

The predetermined shearing zone is dimensioned in such a way that the screw head shears at a given torque which is only a fraction, namely 0.2 to 0.4 times the pulling off force necessary for separating head plate and shank.

In the case of a bone screw known as a M4 screw with a core diameter of 3.1 mm, the torque in the case of a screw made from polylactide is 230 Nmm and in the case of a screw made from polydioxanone 240 Nmm. To permit shearing at the predetermined shearing zone whilst utilizing the invention, the torque in said predetermined shearing zone must be approximately 70 to 72 Nmm. Based on the M4 screw, the desired shearing zone has a diameter of 1.8 mm corresponding to a cross-sectional surface smaller by approximately 70%, based on the core surface of the M4 screw.

Such screws, which are used e.g. in the case of craniofacial malformations, but also for central facial fractures, sagittal clefts of the lower jaw, in lower jaw and cheek bone fractures and wherever it is necessary to fix bone plates, generally have a diameter of 2 to 4 mm and thread lengths of 3 to 10 mm, although diameters of up to 8 mm and thread lengths of up to 100 mm are also possible. Head plates are generally 1 to 3 mm thick and have a diameter 3 mm or more larger than that of the screws.

The inventive bone screws are e.g. used in conjunction with a resorbable fixing system for craniostenosis. In accordance with a bone plate placed on the bones with through-bores appropriate for the bone screw, a hole is drilled in the bones. A thread is then cut into the bone with a tapping attachment, because as a rule the bone screws made from resorbable material do not have an adequate strength to be able to use a self-tapping thread. After making the thread, the inventive bone screw is screwed into the bone with a screwdriver for fixing the bone plate and after stripping the predetermined shearing zone is fixed in such a way that the head plate firmly holds the bone plate on the bone, a depression preferably being provided in the bone plate, which receives the head plate of the inventive bone screw.

If for any reason it is necessary to unscrew the screw, it is easily possible to remove the inventive bone screw using a removal aid, e.g. in the form of an instrument with two projecting pins, which engage in corresponding depressions in the head plate.

It is important for the use of the bone screw according to the invention, that the surgeon can firmly introduce the bone screw in unimpeded manner until the screw head shears and he has the certainty that the screw is now so firmly screwed in that no pulling away forces can occur between the head plate and the shank, which could lead to a premature release of the bone plate, which is also made from resorbable material, from the bone screw made from the same material.

## Claims

1. Bone screw comprising a threaded part, whose shank terminates in one piece in a screw head, characterized in that a head plate (3) is provided between shank (5) and screw head (1) and on whose centre is located said screw head and that the transition zone (2) between the head plate and the screw head is constructed as a predetermined shearing zone, which shears at a torque corresponding to 0.2 to 0.4 times the pulling off force for separating head plate (3) and shank (5).

2. Bone screw according to claim 1, characterized in that the predetermined shearing zone has a cross-sectional surface 60 to 80% smaller than that of the shank.

3. Bone screw according to claim 2, characterized in that two or more vertical bores or recesses (4) for receiving the pins of a box spanner are provided in the outer peripheral region of head plate (3).

4. Bone screw according to claim 1, characterized in that the screw head is constructed as a hexagonal stump.

5. Bone screw according to claims 1 to 3, characterized in that the bone screw is made from a resorbable plastic from the group of polylactides, polyglycolides and/or polydioxanones.

6. Bone screw according to claim 4, characterized in that the torque of the desired shearing point of a M4 screw is in the range 55 to 85 Nmm in the case of a predetermined shearing zone diameter of 1.8 mm.